# EUROPEAN PATENT APPLICATION

(11) **EP 3 643 245 A1**
(43) Date of publication of application: **29.04.2020**
(21) Application number: 18202608.8
(22) Date of filing: 25.10.2018
(51) Int. Cl.: A61B 8/06, A61B 8/12, A61B 8/00

(54) **INTRAVASCULAR DEVICE**

(71) Applicant: Koninklijke Philips N.V., 5656 AG Eindhoven (NL)
(72) Inventor: MUELLER, Manfred, 5656 AE Eindhoven (NL); VAN DER HORST, Arjen, 5656 AE Eindhoven (NL); KUENEN, Maarten Petrus Joseph, 5656 AE Eindhoven (NL)
(74) Representative: de Haan, Poul Erik

(57) **Abstract**

The present invention relates to intravascular flow-sensing. In order to provide flow-sensing with improved accuracy and facilitated handling, an interventional device is provided that comprises an elongated main body with a distal end and an ultrasound transducer arrangement provided on the elongated main body for flow velocity measurement inside an anatomical structure. The elongated main body comprises a base body section and a front tip section. The ultrasound transducer arrangement comprises at least one ultrasound transducer element configured to emit and receive ultrasound waves. The ultrasound transducer arrangement is provided on the base body section proximal to the front tip section, which front tip section provides an offset of the ultrasound transducer arrangement with respect to the distal end. The front tip section is at least partly non-transparent to ultrasound waves. Further, the front tip section is having a minimum extension in the longitudinal direction of the elongated main body of at least five times a diameter of the elongated main body.

## Description

### FIELD OF THE INVENTION

The present invention relates to intravascular flow-sensing, and relates in particular to an interventional device, to an interventional system and to a method for flow-sensing.

### BACKGROUND OF THE INVENTION

Interventional devices that are suitable for insertion into the lumen structures of a patient are used for medical examinations and treatments. For example, intravascular flow measurements are used for clinical applications in cardiology and in peripheral arteries. For example, coronary flow reserve (CFR) is a way to determine whether a stenosis in the coronaries may be flow limiting. Flow measurements can be used in addition or alternatively to pressure measurements for characterizing a stenosis. Coronary flow measurements are also a way to determine microvascular function in a non-obstructive coronary disease. Simultaneous flow and pressure measurements may also be provided to determine pulse wave velocities in blood vessels. For example, US 20110275962 A1 describes the use of ultrasound provided by a catheter. Flow measurements may be considered increasingly important. However, measuring flow may be challenging compared to measuring pressure. For example, flow velocity may vary across the cross-section of the blood vessel and therefore, intravascular flow sensors require more careful maneuvering and positioning.

### SUMMARY OF THE INVENTION

There may thus be a need to provide flow-sensing with improved accuracy and facilitated handling.

The object of the present invention is solved by the subject-matter of the independent claims; further embodiments are incorporated in the dependent claims. It should be noted that the following described aspects of the invention apply also for the interventional device, for the interventional system and for the method for flow-sensing.

According to the present invention, an interventional device is provided that comprises an elongated main body with a distal end. The interventional device also comprises an ultrasound transducer arrangement provided on the elongated main body for flow velocity measurement inside a vascular structure. The elongated main body comprises a base body section, and the elongated main body comprises a front tip section at the distal end. Further, the ultrasound transducer arrangement comprises at least one ultrasound transducer element configured to emit and receive ultrasound waves. The ultrasound transducer arrangement is provided on the base body section proximal to the front tip section, which front tip section provides an offset of the ultrasound transducer arrangement to the distal end. Further, the front tip section is having a minimum extension in the longitudinal direction of the elongated main body of five times a diameter of the elongated main body.

As a result, by integrating the ultrasound transducer into the body of the guidewire away from the tip, the guidewire on both sides of the ultrasound transducer provides improved stability to the ultrasound sensor. This facilitates positioning and alignment of the sensor. Further, the mechanical properties of the tip of the guidewire can be optimized for positioning and do not need to be compromised.

The tip of the guide-wire may be bent a little by the cardiologist to enable better navigation through the vessels. The arrangement of the sensor away from the tip is more convenient for navigation than with a flow sensor directly at the tip. The sensor away from the tip facilitates alignment with respect to the vessel. In an example, the tip can thus be optimized so it can be shaped well and is atraumatic to the vessel wall.

According to an example, the at least one ultrasound transducer element is configured to emit ultrasound waves in a cone-beam form to provide flow sensing data. Further, the cone-beam form covers at least a part of an angular range of between 0° and 60° to the local longitudinal direction of the elongated main body.

In an option, it is provided that the front tip section is at least partly non-transparent to ultrasound waves.

As a result, it is ensured that flow data can be measured.

According to an example, the elongated main body comprises an acoustic window portion adjacent to the at least one ultrasound transducer element. The acoustic window is located between the base body section and the front tip section. Further, the acoustic window portion is at least partly acoustically transparent for the ultrasound waves generated by the at least one ultrasound transducer element.

The acoustic window allows e.g. a continuous outer shape or profile.

According to an example, the ultrasound transducer arrangement comprises an ultrasound wave generating part and an ultrasound beam shaping part. The ultrasound wave generating part comprises the at least one ultrasound transducer element.

Thus, different arrangements are provided for allowing ultrasound tailored to different applications.

According to an example, the ultrasound beam shaping part comprises an ultrasound mirror arrangement that reflects ultrasound waves.

This further provides the options for achieving a variety of different ultrasound beams. The mirror arrangement, e.g., allows to project the ultrasound in a proximal direction, whereas the ultrasound is generated in a distal direction. The mirror thus allows a reversal of the ultrasound beam.

According to an example, the ultrasound beam shaping part comprises an ultrasound absorber that at least partly absorbs ultrasound waves.

It is thus possible to further adapt the emitted ultrasound waves.

According to an example, the ultrasound beam shaping part comprises a support structure. Further, in one option, the support structure comprises i) a centrally arranged core wire section connecting the front tip section to the base body section, wherein the acoustic window section radially surrounds the core wire section. Further, in another option, provided in addition or alternatively, the support structure comprises ii) a centrally arranged cone-shaped core structure with increasing diameter in an ultrasound wave propagation direction.

The support structure thus allows also to arrange cable connections or the like to e.g. other sensors provided in the front tip part.

According to an example, the ultrasound transducer arrangement provides ultrasound waves in two opposite propagation directions; and the ultrasound beam shaping part is a two-part structure having a first sub-part on one side of the ultrasound transducer arrangement and a second sub-part on the other side of the ultrasound transducer. Further, one of the first and second sub-parts comprises an ultrasound reflecting surface.

This provides an increase of emitted ultrasound, but e.g. to one common direction.

According to the present invention, also an interventional system is provided that comprises an interventional device according to one of the preceding examples and a console. The console is configured to control at least one of operation and navigation of the interventional device in a vessel structure.

According to the present invention, also a method for flow-sensing is provided that comprises the following steps:
a) providing an interventional device according to one of the examples above with at least one ultrasound transducer element;
b) generating ultrasound waves by the at least one ultrasound transducer element;
c) receiving reflected ultrasound waves; and
d) determining flow data based on the received ultrasound waves.

According to an aspect, an intravascular guidewire or catheter is provided with an ultrasound flow sensor, the sensor being offset from the tip by e.g. at least 1 cm, such as 2 cm or more. The part of the device that is distal from the ultrasound sensor is made at least partly from an acoustic window material that is at least nearly transparent for the ultrasound waves from the transducer. The intravascular guidewire or catheter may end in a functional tip.

According to an aspect, an acoustic window section that is at least partly transparent to ultrasound waves is connecting the tip to rest of the guidewire. In addition, the acoustic window section enables the integration of ultrasound reflecting parts, to reshape the ultrasound waves and e.g. improve the size of the sensing cone of the transducer or the signal-to-noise ratio of the device.

In an example, the part of the device that is distal from the ultrasound sensor can also contain elements that reflect the ultrasound waves and/or otherwise reshape the ultrasound waves. An example of the latter could be a curved mirror cone that increases the sensing angle of the ultrasound beam cone. The part of the device that is distal from the ultrasound sensor can also contain elements that absorbs ultrasound wave energy to avoid spurious reflections.

According to an aspect, the guidewire comprises three main parts: the proximal part, the acoustic window part, and the tip part. In an example, the proximal part (or shaft) has the ultrasound transducer at its end. The acoustic window part is primarily made from a material that is substantially transparent to the acoustic waves from the ultrasound transducer. Ideally this material is also impedance matched to blood for the acoustic waves from the ultrasound transducer. Suitable exemplary materials for acoustical window are, Polymethylpentene (e.g. TPX® by Mitsui Chemicals), biaxially-oriented polyethylene terephthalate (e.g. Mylar® by Dupont Tejjin Films), Polyether block amide with different grades (e.g. medical grades PEBAX® by Arkema). Therefore, a large percentage of the ultrasound waves from the transducer as well as the waves reflected from the blood will simply pass through the acoustic window part, therefore ensuring the functionality of the proximal part. The acoustic window can also contain parts that absorb the waves from the ultrasound transducer. For example, an absorber (e.g. HAM A, AptFlex F28 by Precision Acoustics), is provided where the acoustic window connects to the tip part to avoid or at least reduce reflections from the tip. In addition, the acoustic window part ensures the mechanical connection between the proximal part and the tip part. If necessary, the mechanical connection can be improved by using an additional (metal) housing with windows for the ultrasound waves. The tip part may be similar to the tip of a standard cardiology guidewire. It may contain all such parts to tailor the mechanical properties of a guidewire tip such as spring coils, hypotubes, an atraumatic tip, a tapered core and the like.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of an interventional device with an ultrasound transducer arrangement.
Fig. 2 shows a schematic cross-section of a tip section of an example of the interventional device.
Fig. 3 shows a schematic cross-section of a tip section of another example of the interventional device with an ultrasound mirror arrangement.
Fig. 4 shows a schematic cross-section of a tip section of a further example of the interventional device with a further example of the ultrasound mirror arrangement.
Fig. 5 shows a schematic cross-section of a tip section of a still further example of the interventional device with an ultrasound mirror arrangement with concave surface portions.
Fig. 6 shows a schematic cross-section of a tip section of another example of the interventional device with a core wire support structure.
Fig. 7 shows a schematic cross-section of a tip section of a further example of the interventional device with a cone-beam shaped support structure.
Fig. 8 shows a schematic cross-section of a tip section of a still further example of the interventional device with an ultrasound transducer generating waves in two directions and a mirror structure to emit the ultrasound waves in one common direction.
Fig. 9 shows an example of an interventional system.
Fig. 10 shows steps of an example of a method for flow-sensing.

### DETAILED DESCRIPTION OF EMBODIMENTS

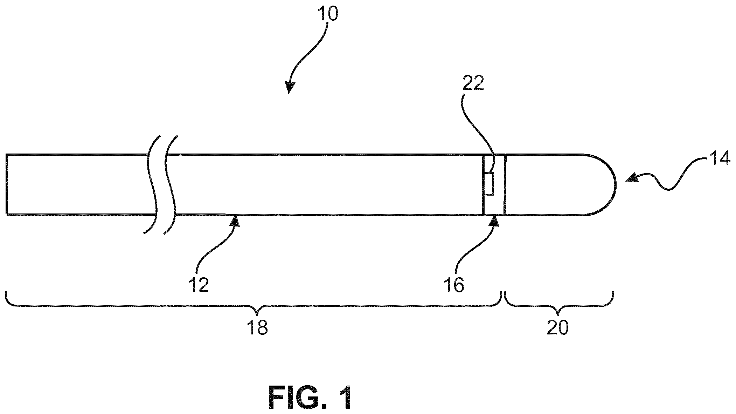

Fig. 1 schematically shows an example of an interventional device 10. The interventional device 10 comprises an elongated main body 12 with a distal end 14. The interventional device 10 further comprises an ultrasound transducer arrangement 16 provided on the elongated main body 12 for flow velocity measurement inside a vascular structure. The elongated main body 12 comprises a base body section 18. The elongated main body 12 further comprises a front tip section 20 at the distal end 14. The ultrasound transducer arrangement 16 comprises at least one ultrasound transducer element 22 configured to emit and receive ultrasound waves. The ultrasound transducer arrangement 16 is provided on the base body section 18 proximal to the front tip section 20, which front tip section 20 provides an offset of the ultrasound transducer arrangement 16 to the distal end 14. The front tip section 20 is having a minimum extension in the longitudinal direction of the elongated main body 12 of at least five times a diameter of the elongated main body 12.

The front tip section 20 may comprise a flexible body with a flexible support (see also below).

In an option, the front tip section 20 is at least partly non-transparent to ultrasound waves.

In an example, the front tip section 20 has a minimum length of 1 cm; in another example, a minimum of 2 cm is provided.

The term "non-transparent" (or "opaque") relates to a characteristic of the front tip section that blocks at least half of the ultrasound waves from being transmitted through the front tip section if generated behind the front tip section. In an example, a minimum of 80% or 90% is blocked.

The ultrasound waves are provided in a range that is commonly applied with intravascular ultrasound transducer arrangements in medical technology.

The elongated main body 12 can also be referred to as longitudinal elongated main body. In an example, the distal end 14 is configured for navigating in a vascular structure.

The distal end 14 is provided by a first end of the elongated main body 12. A second end of the elongated main body 12 is then provided at the opposite end of the elongated main body 12. Further, a proximal section is provided at the second end.

In an example, the elongated main body 12 is provided with a functional tip at the distal end 14 for facilitating navigation within a vascular structure. The functional tip may be provided as a rounded or tapered end section for a trauma-free (or atraumatic) navigation.

In an example, the ultrasound transducer arrangement 16 is configured for flow measurement inside the vascular structure. The front tip section 20 is provided between the ultrasound transducer arrangement 16 and the distal end 14. The front tip section 20 can also be referred to as front tip part or distal portion.

In an example, the elongated main body 12 comprises a main shaft section and a transducer section between the main shaft section and the front tip section.

The term "provided on" refers to an arrangement where the ultrasound transducer arrangement 16 is mounted to the elongated main body 12. Thus, the ultrasound transducer arrangement 16 is attached and carried by the elongated main body 12. In an example, the ultrasound transducer arrangement 16 is part of the elongated main body 12.

In another example, the ultrasound transducer arrangement 16 is configured to provide a single viewing or detection direction.

In an example, the ultrasound transducer arrangement 16 is configured to provide a viewing or detection direction aligned with a flow direction in the particular vessel.

In another example, the ultrasound transducer arrangement 16 is configured to provide two opposite viewing or detection directions.

The interventional device 10 is provided to be partly inserted into an object (e.g. an anatomical structure such as vessel or organ). The part that is inserted first is referred to as distal part, i.e. a part further away from the user. The part that is used for handling and navigating is referred to as proximal part, i.e. a part closer to the user.

The interventional device 10 as described above is applicable, for example, in the field of intravascular flow measurement and intravascular pulse-wave velocity measurement.

The at least one ultrasound transducer element 22 transmits pulsed ultrasound waves and receives reflections of those pulses from moving blood cells, e.g. allowing calculation of blood velocity using the Doppler principle. For accurate flow measurements, the transducer is placed in the center of and aligned with the blood vessel. As the guidewire offers stabilization to the sensor being arranged between two sections of the guidewire, the positioning is facilitated. Also in coronary vessels, the stabilization is improved and it is avoided that the sensor flops around in an uncontrolled way during the cardiac cycle due to the movements of the vessel. Furthermore, it is avoided frontal contact of the flow measurement sensor with the vessel wall or organ (e.g. heart wall).

As indicated above, the ultrasound sensor of a flow measure guidewire is offset away from the tip without compromising functionality. The position of the offset sensor is then stabilized by the guidewire on both sides of the sensor, leading to a more stable measurement position and easier sensor alignment. Also, the mechanical properties of the tip of the guidewire are optimized, since there is no longer the need to include the sensor. This further facilitates sensor placement.

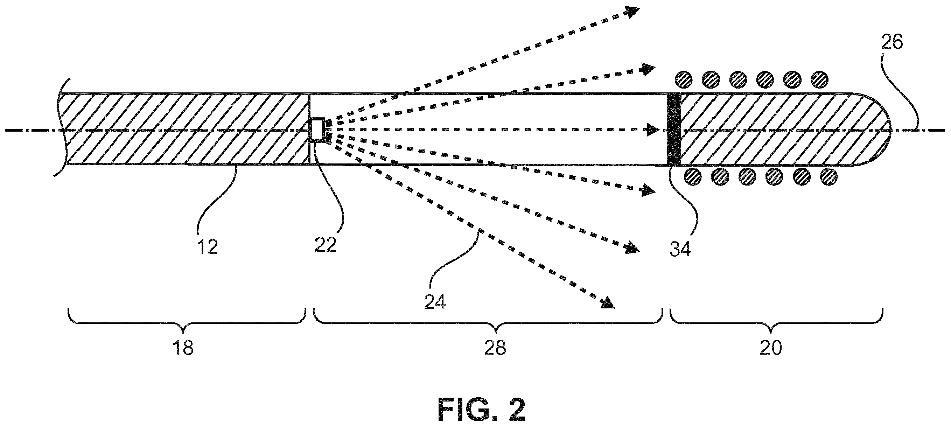

Fig. 2 shows an example, where the at least one ultrasound transducer element 22 is configured to emit ultrasound waves 24 in a cone-beam form to provide flow sensing data. The cone-beam form covers at least a part of an angular range of between 0° and 60° to a longitudinal direction 26 of the elongated main body 12.

The cone-beam is provided to cover at least a part of a cone-shaped range with a cone angle of 120°, i.e. from -60° to +60. It is noted that 0° refers to a direction parallel to the local longitudinal direction 26 of the elongated main body 12, and 60° refers to a respective angle in relation to the local longitudinal direction 26 of the elongated main body 12. In an example, the range covers parallel directions and also anti-parallel directions. The range of 0° to 60° refers to the extension of the beam in a radial direction around the elongated main body 12, i.e. to a cone-shaped range.

It is noted that since the main body may be provided flexible, the longitudinal direction may change along e.g. a curved main body. The term "local" longitudinal direction thus refers to the portion of the main body where the ultrasound sensor is located.

As an example, a range of 0° to maximum 45° is provided. In an example, a range of 0° to maximum 30° is provided. In a further example a range of 0° to 15°.

Hence, the ultrasound is emitted such that flow information of blood particles moving along the vessel can be retrieved.

For example, a part of the waves may be directed perpendicular to the elongated main body, and thus more or less perpendicular to a vessel in which the sensor is arranged, but these waves do not provide flow information.

In an example, the at least one ultrasound transducer element 22 is configured to emit ultrasound waves in a direction having a vector component in a longitudinal direction of the elongated main body. In other words, ultrasound waves are emitted in a direction within a range of parallel to the elongated main body and angled or transverse to the elongated main body, but not perpendicular. The ultrasound wave will have an inclination from the perpendicular such that measurements of the flow in flow direction are possible.

The ultrasound waves having a vector component in a longitudinal direction of the elongated main body 12 provide a viewing direction in flow direction of a vessel.

In an example, the at least one ultrasound transducer element is configured to emit ultrasound waves along the longitudinal direction 26 of the elongated main body 12.

In an example, the at least one ultrasound transducer element 22 is configured to emit ultrasound waves in a cone-beam form in the longitudinal direction of the elongated main body 12 to provide flow sensing data.

As shown in Fig. 2 as an option, the elongated main body comprises an acoustic window portion 28 adjacent to the at least one ultrasound transducer element 22. The acoustic window portion 28 is located between the base body section 18 and the front tip section 20. The acoustic window portion 28 is at least partly acoustically transparent for the ultrasound waves generated by the at least one ultrasound transducer element 22.

The acoustic window structurally connects the front tip section 20 with the base body section 18.

In an example, the acoustic window portion 28 is provided acoustically transparent in all directions, i.e. in 360 degrees around the longitudinal axis of the device.

In an example, the ultrasound source is provided proximal to the acoustic window portion. In another example, the acoustic window section connects the front tip section with the base body section.

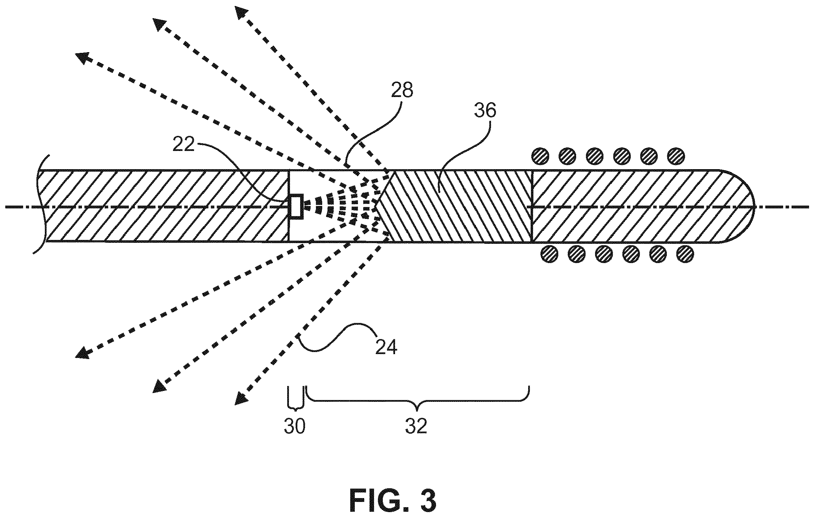

Fig. 3 shows an option that the ultrasound transducer arrangement 16 comprises an ultrasound wave generating part 30 and an ultrasound beam shaping part 32. The ultrasound wave generating part 30 comprises the at least one ultrasound transducer element 22. The ultrasound beam shaping part 32 comprises the acoustic window portion.

The term transducer arrangement 16 thus relates to having the transducer arrangement 16 between the base body section 18 and the front tip section 20. The transducer arrangement 16 can then be further subdivided according to its function in the part that generates the beam, and another part that shapes the beam.

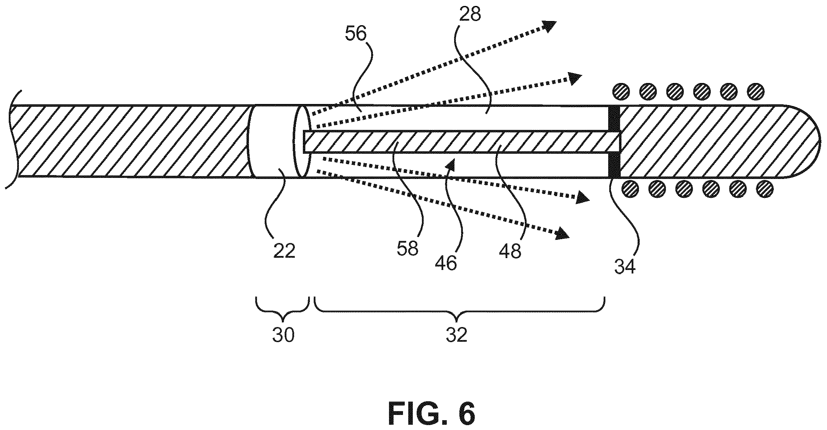

Shown as an option e.g. in Fig. 2 or Fig. 6, an ultrasound absorber 34 may be provided that at least partly absorbs ultrasound waves. The absorber absorbs ultrasound wave energy to avoid spurious reflections at e.g. a part of the device that is distal from the ultrasound sensor.

The ultrasound beam shaping part 32 comprises an ultrasound mirror arrangement 36 that reflects ultrasound waves. The mirror arrangement 36, also referred to as mirror, is provided to guide and/or shape the ultrasound waves emitted by the ultrasound transducer toward the anatomy comprising the particles (e.g. red blood cells) and also to guide, i.e. re-direct the incoming ultrasound waves reflected (e.g. echo) from particles toward the ultrasound transducer in order to measure the reflected ultrasound waves.

In the example of Fig. 3, the guidewire uses a proximal part (to the left, not shown) and a tip part (to the right). The acoustic window part 28 is provided which includes elements, i.e. the mirror arrangement 36, for reflecting the ultrasound waves from the transducer as well as ultrasound waves reflected from the blood.

For example, the reflective element has the shape of a cone. Furthermore, other shapes are also provided. The geometry of the reflective element can be amended and thus optimized (together with the transducer's beam profile) so as to achieve an optimal resulting beam profile.

For example, the ultrasound waves can be generated towards a first direction and can then be reflected in an opposite direction, as indicated in Fig. 3.

The cone can be made of any metal or any other material that reflects ultrasound waves. Using a reflective element results in that the length of the acoustic window can be reduced. Also, more of the ultrasound energy from the transducer can be used, because energy is not lost at the tip.

In an example, the ultrasound beam shaping part is also referred to as a wave guiding and distributing part.

Fig. 3 shows the mirror arrangement 36 cone-shaped or conus-shaped or tapered.

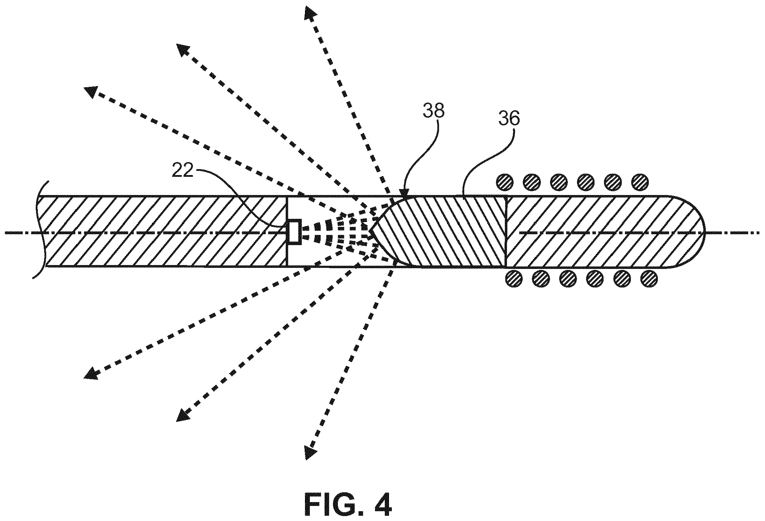

Fig. 4 shows a further example where portions 38 of the mirror arrangement 36 are chamfered or slanted. Thus, the ultrasound cone is widened. As an example, the outer edges may be chamfered or slanted. In other words, in Fig. 4, the mirror element is curved so that the opening cone of the ultrasound wave coming from the transducer is increased. This decreases the sensitivity of the sensor to misalignment with the vessel wall, since a larger spatial angle is sampled. However, the divergent beam will decrease the signal-to-noise-ratio. A pointed tip may be provided to avoid direct backreflection of the ultrasound waves from the mirror arrangement toward the ultrasound transducer.

With the transducer configured as described above and below, at least one side of the beam is provided that is aligned with the flow because of circular symmetry.

Further, a focused mirroring effect may be provided, by e.g. a parabolic (or similar) shape of the ultrasound mirror surface. Focusing provides a good signal to noise ratio, e.g. against other undesired components, e.g. the part of the beam that goes wide off the ultrasound mirror (and hence detects forward flow). This improves the accuracy and reliability of the velocity estimation. Further, focusing also provides a better definition of the Doppler angle: With the proposed arrangement, the angle between the ultrasound beam and the flow direction (also known as the Doppler angle) is increased. By focusing the beam, the distribution of Doppler angles is concentrated around one value, facilitating a direct calculation of the velocity.

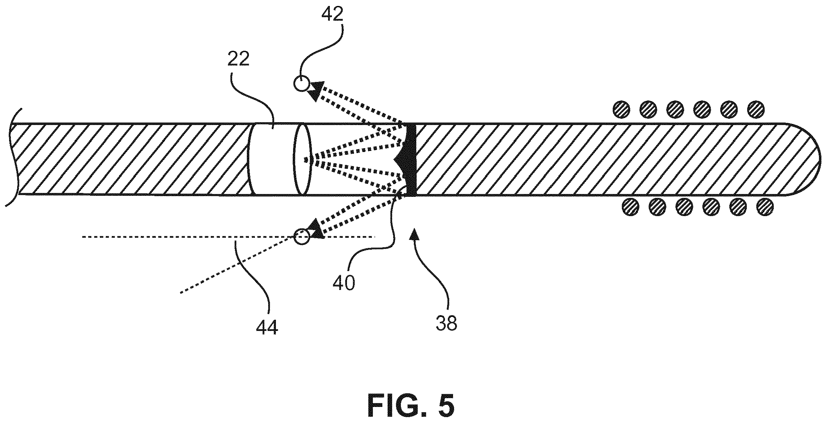

Fig. 5 shows a further example where the ultrasound mirror arrangement 38 comprises at least one concave reflecting surface portion 40 that provides focused ultrasound waves. A focal point 42 is indicated with a small circle. As indicated, the focused ultrasound waves provide a focal point in a predetermined location.

The focused ultrasound waves provide a Doppler angle 44 downstream of the focal point 42.

In an example, the ultrasound beam shaping part comprises an ultrasound mirror that reflects ultrasound waves in order to provide a cone beam or ultrasound waves. It is noted that the concave reflecting surface portions are provided as an option.

In another option, not shown in detail, the ultrasound mirror arrangement comprises at least one convex reflecting surface portion that increases the opening angle of the ultrasound beam, i.e. that provide further distributed ultrasound waves.

Fig. 6 shows as an option that the ultrasound beam shaping part comprises a support structure 46. For example, the support structure 46 comprises a centrally arranged core wire section 48 connecting the front tip section to the base body section. The acoustic window section radially surrounds the core wire section.

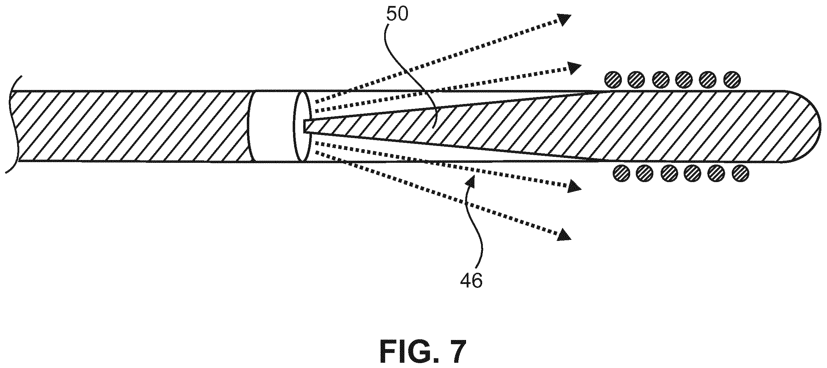

Fig. 7 shows as another option that the ultrasound beam shaping part comprises a centrally arranged cone-shaped core structure 50 with increasing diameter in an ultrasound wave propagation direction.

As shown in Fig. 6 as an option, the acoustic window portion 28 may comprise acoustically transparent sections 56 and acoustically opaque or non-transparent sections 58. For example, the opaque or non-transparent sections 58 may be provided by wire connections for connecting further sensors arranged in the front tip portion or support structures.

In an example (see e.g. Fig. 6), a core wire passes from the proximal part to the distal portion. This improves the stability and other mechanical properties of the guidewire as well as the manufacturability. In this example, the ultrasound transducer surrounds the core. This can be done by using a single ring-shaped or annular (donut-shaped) transducer or by using multiple smaller transducers (e.g. CMUT transducers) disposed surrounding the core.

In an example, the support structure is acoustically opaque.

Fig. 7 shows a schematic cross-section of a tip of a further example of an interventional device with a cone-beam shaped support structure. An ultrasound absorber is not required by changing the core wire into a conically shaped rigid core. This has the benefit that the acoustic energy is no longer absorbed (and consequently not effectively used), but reflected of the conical shape and therefore contributing to the transducer's beam profile. This will improve the signal-to-noise ratio.

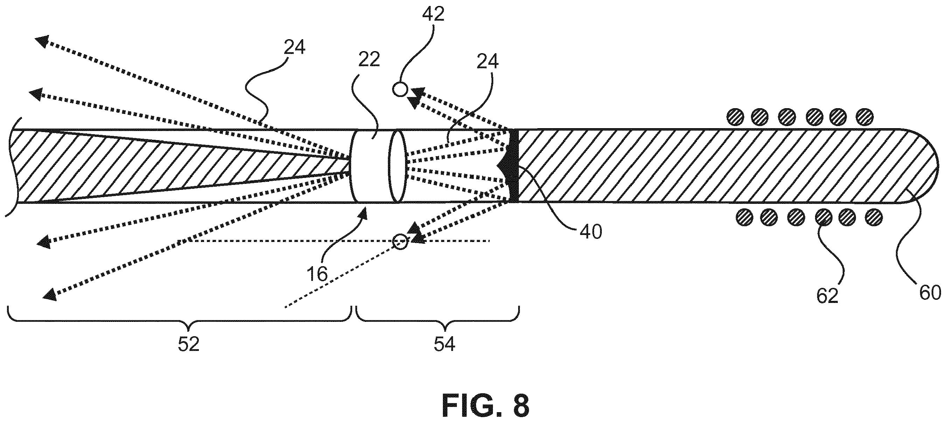

Fig. 8 shows an example where the ultrasound transducer arrangement provides ultrasound waves in two opposite propagation directions. As an example, the ultrasound waves 24 are generated to the right side and the left side, i.e. in distal and proximal direction. The ultrasound beam shaping part is a two-part structure having a first sub-part 52 on one side of the ultrasound transducer arrangement 16 and a second sub-part 54 on the other side of the ultrasound transducer. One of the first and second sub-parts comprises an ultrasound reflecting surface, such as the concave reflecting surface portion 40. Other forms and shapes for the reflecting surface are also provided. The generation of ultrasound in two opposite directions is also referred to as bi-directional ultrasound generation. The ultrasound transducer creates acoustic energy in a bi-directional sense, i.e. both forward and backwards along the longitudinal direction of the elongated main body. As a result, a larger part of the transmitted acoustic energy is used to assess the velocity, potentially increasing the overall signal-to-noise ratio of the system.

In an example, the ultrasound reflecting surface is provided with at least one concave mirror surface or with a convex mirror surface.

In a further example, the ultrasound reflecting surface is provided with a cone beam shaped mirror.

As indicated in Fig. 8 and mentioned above, the front tip section may comprise a flexible body section part 60 with a flexible support structure 62. In an example, the support structure is provided as a coil-shaped spring structure, such as a spiral shaped spring. The support structure can form a shaped tip or can be used to shape the tip. For example, the spring coil structure is provided as a spiral spring element at least partly enclosing a flexible (e.g. elastic, bendable) main body structure.

It is noted that although the flexible support structure 62 is shown in Figs. 2 to 8, the flexible support structure 62 is only provided as an option. Examples are provided where the flexible support structure 62 is omitted.

In an example, the at least one ultrasound transducer element 22 is provided integrated in the elongated main body 12 such that the elongated main body 12 forms a continuous shaft portion.

In another example, the ultrasound transducer arrangement provides ultrasound waves in two opposite propagation directions and the ultrasound beam shaping part is a two-part structure having a first sub-part on one side of the ultrasound transducer arrangement and a second sub-part on the other side of the ultrasound transducer. Further, ultrasound waves are generated to propagate in two opposite directions.

In an option, the at least one ultrasound transducer element is provided as at least one of the group of a piezo-electric transducer and a CMUT-transducer and a PMUT transducer.

In an example, a single piezo-electric transducer is provided.

In another example, a plurality of piezo-electric transducers is provided.

In an example, a single CMUT-transducer (capacitive micro-machined ultrasonic transducer) is provided.

In an example, a single PMUT-transducer (piezoelectric micro-machined ultrasonic transducer) is provided.

In another example, a plurality of CMUT-transducers is provided.

In a further example, at least one piezo-electric transducer is arranged in combination with at least one CMUT-transducer.

It is noted that the above-described arrangements of the ultrasound source and the guiding parts are provided in the shown and described arrangements in relation to the interventional device direction defined by the distal end as the end that is inserted in an object first. In a further example, the arrangements are provided in the opposite arrangements in relation to the interventional device direction.

As an option, not shown in detail, the interventional device is an intravascular guidewire or catheter.

In an example, flow and pressure sensors are provided on the guidewire. The guidewire may be provided as a flexible wire, e.g. with a floppy characteristic outside a vessel structure, but stiff enough to be steered through a vasculature structure.

In any of the embodiments of the interventional devices disclosed, suitable exemplary materials for acoustical window may be chosen from, Polymethylpentene (e.g. TPX® by Mitsui Chemicals), biaxially-oriented polyethylene terephthalate (e.g. Mylar® by Dupont Tejjin Films), Polyether block amide with different grades (e.g. medical grades PEBAX® by Arkema).

In any of the embodiments of the interventional devices disclosed, a face of the ultrasound transducer, emitting the ultrasound waves for flow velocity measurement of fluid comprising particles streaming through a vessel or an organ, can be arranged to be substantially perpendicular to longitudinal axis 26 of the interventional device. This is particularly advantageous in the manufacturing process, e.g. not necessitating curved face ultrasound transducer.

In any of the embodiments of the interventional devices disclosed, wherein the acoustic window is applicable, the acoustic window material may be filling the space between the base body section 18 and the front tip section 20 remaining besides other functional structures (e.g. mirrors, acoustic absorber, core wire structure), and the outer surface of the acoustic window is arranged for a smooth transition between the outer diameter of the base body section 18 and the front tip section 20. Alternatively, the acoustic window may comprise a lumen, filled with a fluid (e.g. saline) through a lumen within the base body section 18 arranged in fluid communication with the lumen of the acoustic window.

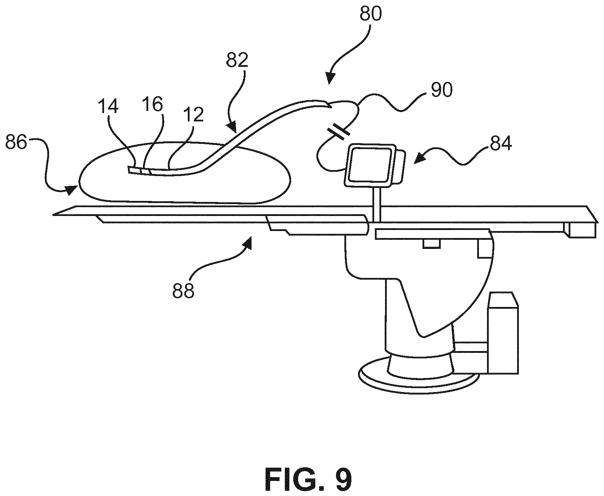

Fig. 9 shows an example of an interventional system 80. The interventional system 80 comprises an interventional device 82 according to one of the preceding examples and a console 84. The console 84 is configured to ascertain the flow velocity from the measurement data provided by the ultrasound sensor of the interventional device. The console 84 may further be configured to control at least one of operation and navigation of the interventional device in a vessel structure. The interventional device 82 is shown as a guidewire partly inserted into a subject 86. The subject 86 may be arranged on a patient support 88. The interventional device 82 is connected to the console with data connection 90, either wireless or wire bound.

The term "subject" may also be referred to as individual. The "subject" may further also be referred to as patient, although it is noted that this term does not indicate whether any illness or disease is actually present with the subject.

In an example, the interventional device is provided as a guidewire. Further, a handling section is releasably attached to a proximal end of the interventional device; and the handling section is configured to control navigation of the interventional device in a vessel structure.

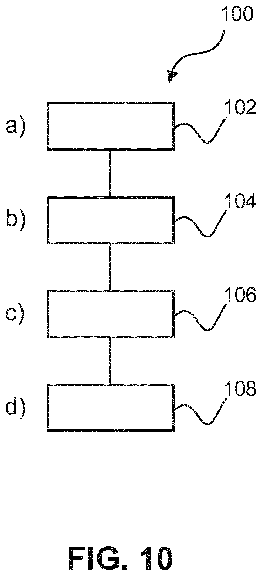

Fig. 10 shows steps of an example of a method 100 for flow-sensing.

In a first step 102, also referred to as step a), an interventional device according to one of the examples above with at least one ultrasound transducer element is provided.

In a second step 104, also referred to as step b), ultrasound waves are generated by the at least one ultrasound transducer element.

In a third step 106, also referred to as step c), reflected ultrasound waves are received.

In a fourth step 108, also referred to as step d), flow data is determined based on the received ultrasound waves.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An interventional device (10) comprising:
- an elongated main body (12) with a distal end (14); and
- an ultrasound transducer arrangement (16) provided on the elongated main body for flow velocity measurement inside an anatomical structure;
wherein the elongated main body comprises a base body section (18), and wherein the elongated main body comprises a front tip section (20) at a distal portion;
wherein the ultrasound transducer arrangement comprises at least one ultrasound transducer element (22) configured to emit and receive ultrasound waves;
wherein the ultrasound transducer arrangement is provided on the base body section proximal to the front tip section; and
wherein the front tip section is having a minimum extension in a longitudinal direction of the elongated main body of at least five times a diameter of the elongated main body.

2. Interventional device according to claim 1, wherein the at least one ultrasound transducer element is configured to emit ultrasound waves in a cone-beam form to provide flow sensing data;
wherein the cone-beam form covers at least a part of an angular range of between 0° and 60° to a local longitudinal direction of the elongated main body; and
wherein the front tip section is at least partly non-transparent to ultrasound waves.

3. Interventional device according to one of the preceding claims, wherein the elongated main body comprises an acoustic window configured to structurally connect the base body section and the front tip section; and
wherein the acoustic window portion is substantially transparent for the ultrasound waves generated by the at least one ultrasound transducer element.

4. Interventional device according to one of the preceding claims, wherein the ultrasound transducer arrangement comprises an ultrasound wave generating part (30) and an ultrasound beam shaping part (32);
wherein the ultrasound wave generating part comprises the at least one ultrasound transducer element; and
wherein the ultrasound beam shaping part comprises the acoustic window portion.

5. Interventional device according to claim 4, wherein the ultrasound beam shaping part further comprises an ultrasound mirror arrangement (36) that reflects ultrasound waves.

6. Interventional device according to claim 5, wherein the ultrasound mirror arrangement comprises at least one concave reflecting surface portion (40) that provides focused ultrasound waves.

7. Interventional device according to claim 5 or 6, wherein the ultrasound mirror arrangement comprises at least one convex reflecting surface portion that increases the opening angle of the ultrasound beam.

8. Interventional device according to one of the preceding claims, wherein the ultrasound beam shaping part comprises an ultrasound absorber (34) that substantially absorbs ultrasound waves.

9. Interventional device according to one of the preceding claims, wherein the ultrasound beam shaping part comprises a support structure (46); and
wherein the support structure comprises:
i) a centrally arranged core wire section (48) connecting the front tip section to the base body section, wherein the acoustic window section radially surrounds the core wire section; or
ii) a centrally arranged cone-shaped core structure (50) with increasing diameter in an ultrasound wave propagation direction.

10. Interventional device according to one of the preceding claims, wherein the ultrasound transducer arrangement provides ultrasound waves in two opposite propagation directions; and the ultrasound beam shaping part is a two-part structure having a first sub-part (52) on one side of the ultrasound transducer arrangement and a second sub-part (54) on the other side of the ultrasound transducer; and
wherein one of the first and second sub-parts comprises an ultrasound reflecting surface.

11. Interventional device according to one of the preceding claims, wherein the front tip section comprises a flexible body section part with a flexible support structure.

12. Interventional device according to one of the preceding claims, wherein the at least one ultrasound transducer element is provided as at least one of the group of a piezoelectric transducer, a CMUT-transducer and a PMUT transducer.

13. Interventional device according to one of the preceding claims, wherein the interventional device is an intravascular guidewire or catheter.

14. An interventional system (80) comprising:
- an interventional device (82) according to one of the preceding claims; and
- a console (84);
wherein the console is configured to ascertain a flow velocity from measurement data provided by the ultrasound transducer arrangement.

15. A method (100) of flow-sensing comprising the following steps:
a) providing (102) an interventional device according to one of the claims 1 to 13 with at least one ultrasound transducer element or a system according to claim 14;
b) generating (104) ultrasound waves by the at least one ultrasound transducer element;
c) receiving (106) reflected ultrasound waves; and
d) determining (108) flow data based on the received ultrasound waves.
